# EUROPEAN PATENT APPLICATION

(11) **EP 1 415 549 A1**
(43) Date of publication of application: **06.05.2004**
(21) Application number: 02024313.5
(22) Date of filing: 01.11.2002
(51) Int. Cl.: A23L 2/52, A61K 31/355, A61K 35/78

(54) **Synergistic antioxidant combination of tocols and polyphenols**

(71) Applicant: Galileo Laboratories, Inc., Santa Clara, CA 95054 (US)
(72) Inventor: Miller, Guy, San Jose, CA 95112 (US); Flaim, Stephen F., San Diego, CA 92130 (US)
(74) Representative: Barz, Peter, Dr.

(57) **Abstract**

A source of α-tocol or an acceptable salt or ester thereof acts synergistically with an antioxidant source comprising polyphenols, to effect suppression of LDL oxidation in serum. The combination of these two antioxidant sources has wide-ranging applications in treatment of medical conditions arising from free radical generation, including arteriosclerosis, rheumatism and cancer.

## Description

### Field of the Invention

The present invention relates to compositions comprising a combination of at least one source of non-α-tocols (tocotrienol and/or tocopherol), with an antioxidant source comprising polyphenolic compounds. Due to synergistic effects, such compositions are useful in treating or preventing disorders caused by free radicals, such as those relating to ischemia, including coronary heart disease, cancer, inflammation, and including rheumatism.

### Background of the Invention

There is ample evidence suggesting that diets rich in fruit and vegetables protect against the development of chronic illnesses such as Coronary Heart Disease (CHD) and cancer, which are the major killer diseases among affluent populations. It has been recognized for some time that these beneficial effects are due, at least in part, to the antioxidant components of these foodstuffs, which can inhibit cellular damage by free radicals. In the body, free radical damage arises due to reactive oxygen species (ROS) being formed endogenously within the tissues, and also as a result of exposure to radicals arising from cigarette smoke, ionizing radiation, air pollution and other environmental insults. These free radicals have the potential to interact with biological molecules such as proteins, lipids and DNA, and they are implicated in the aetiology of diverse diseases.

Antioxidants are likely to come into play in many aspects of multi-factorial disease conditions such as cancer or CHD, so it is difficult to establish how their primary effects are exerted. However, in the case of the process of atherogenesis a clear link has been demonstrated between oxidation of low density lipoprotein (LDL) and the formation of occlusive plaque in the arteries. Vitamin E (α-tocopherol) consumed as part of the diet is naturally present in the blood, where it acts to spare the LDL from oxidation. In vitro experiments have established that other natural sources of antioxidants, such as green tea, can also significantly retard the oxidation of LDL. In vivo studies have confirmed the *in vitro* tests, showing that increasing dietary intakes of some antioxidants, such as Vitamin E, can increase the resistance of LDL to oxidation and thereby reduce lesions in the arteries.

The main antioxidants from dietary sources are vitamins E (α-tocopherol) and C (ascorbic acid), the carotenoids, and polyphenols. Due to an increase in public awareness of the importance of these micronutrients, it has become commonplace for people to supplement their diets with natural or synthetic antioxidant sources. However, in spite of this general shift in favor of healthier eating patterns the incidence of CHD and cancer continues to be of concern.

It is clearly easier to encourage the use of dietary antioxidant supplements than to induce radical changes in the diet. But dietary supplements have their own drawbacks, particularly since there are detrimental side effects associated with high doses. Recently, for instance, the US Institute of Medicine advised that the upper limit of intake of vitamin E should be set at 1000mg α-tocopherol per day, because of the risk of stroke and uncontrolled bleeding due to the anti-coagulant properties of this nutrient. β-carotene has actually been linked to an increase in the incidence of cancer. Thus, at the maximum safe dosage the antioxidant effect may be less than optimal.

Consequently, there is a need for new antioxidant products, which can be provided in the form of a food or as a dietary supplement, and which are potent in preventing CHD, cancer and other diseases associated with free radical generation, at dosages which do not pose health risks.

### Summary of the Invention:

According to a first aspect of the present invention there is provided a composition comprising a mixture of:
(a) a source of non-α-tocol or an acceptable salt or ester thereof comprising greater than 2% by weight non-α-tocol or an acceptable salt or ester thereof; and
(b) polyphenol or a or an acceptable salt or ester thereof ,
with the proviso that when (b) comprises citrus flavonoids, (a) comprises non-α-tocopherol or an acceptable salt or ester thereof.

In a second aspect of the invention there is provided a composition comprising in a daily dosage:
(a) at least 5mg of a non-α-tocopherol or a or an acceptable salt or ester thereof or a mixture of γ-tocopherol or an acceptable salt or ester thereof and δ-tocopherol or an acceptable salt or ester thereof; and
(b) polyphenol or a or an acceptable salt or ester thereof.

In a further aspect of the invention there is provided a food or beverage product comprising:
(a) a source of non-α-tocol or an acceptable salt or ester thereof comprising greater than 2% by weight γ-tocopherol or an acceptable salt or ester thereof or δ-tocopherol or an acceptable salt or ester thereof; and
(b) an antioxidant source comprising polyphenol or an acceptable salt or ester thereof, and optionally one or more of carbohydrate, fat, fibre and protein.

In one aspect the compositions disclosed herein are derived from sources of non-α-tocols and sources of polyphenols. Such sources may be natural sources or non-natural sources, that is sources manufactured by man.

In another aspect the compositions disclosed herein are modified sources of non-α-tocols and modified sources of polyphenols.

In a further aspect, the compositions disclosed herein are compositions which are enriched in polyphenols composed with natural sources of polyphenols.

Each individual aspect of the compositions may be combined with each other individual aspect of the compositions. For example, a natural source of non-α-tocol may be combined with a modified source of polyphenol, etc. Modifications of sources include chemical or physical modification, for example, derivatives of naturally occurring compounds such as salts or esters or sources obtained after physical treatment such as extract, solutions, mixtures, etc.

In yet another aspect of the invention there is provided a pharmaceutical composition or dietary supplement comprising the composition of the invention as claimed, with a pharmaceutically acceptable carrier.

In another aspect of the invention there is provided use of a composition according to the invention as claimed, as a scavenger of free radicals.

In a further aspect of the invention there is provided a composition according to the invention as claimed, for use as a medicament.

In yet another aspect of this invention there is provided use of a composition according to the invention as claimed, in the manufacture of a medicament or nutritional formulation for the prevention or treatment of a disease condition or corporal damage associated with the generation of free radicals.

In another aspect of the invention there is provided a kit comprising:
(a) a source of non-α-tocol or an acceptable salt or ester thereof, preferably comprising greater than 2% by weight non-α-tocol or an acceptable salt or ester thereof; and
(b) an antioxidant source comprising polyphenol or an acceptable salt or ester thereof, for separate, sequential or simultaneous administration.

### Detailed Description of the Invention

It has surprisingly been found that the antioxidant properties of a combination of a tocol source comprising non-α-tocotrienol and/or non-α -tocopherol, particularly δ-tocopherol or γ-tocopherol, with an antioxidant source comprising polyphenols, are more than additive, i.e. we have observed a synergistic effect in preventing oxidation and cell death in cell oxidation and inflammation models, when exemplary non α-tocols, such as γ-tocopherol or δ-tocopherol, are combined with exemplary polyphenols, such as flavonoids, as detailed in Example 1.

As a result of this synergy, it is possible to obtain an equivalent or improved antioxidant effect at lower dosages compared with conventional antioxidant products. This has the advantage that a powerful antioxidant effect can be achieved *in vivo* while avoiding the need to ingest any single antioxidant at levels that could threaten health. In addition, the maximum antioxidative benefit obtainable is superior to the maximum benefit obtainable through use of these antioxidants separately.

Tocopherols and tocotrienols (sometimes collectively known as vitamin E, or "tocols") are fat-soluble biological membrane components that are structurally-related, having the same aromatic chromanol "head". Whereas tocopherols have a saturated isoprenoid side chain, tocotrienol side chains are unsaturated. Tocols are found in α (alpha), β (beta), γ (gamma) and δ (delta) forms, classified according to the position of substitution on the phenol ring. In the context of the present invention the generic term "tocol" is used to refer to any isomer of tocotrienol or tocopherol or salt form thereof, or any mixture (racemic or otherwise) of such molecules. "δ-tocols" are a sub-group of these tocols, i.e. those having a δ-monomethyl group on the benzene ring of the chroman moiety, and this term is intended to refer to any isomer of δ-tocotrienol or δ-tocopherol or salt form thereof, or any mixture (racemic or otherwise) of such molecules. Similarly, "γ-tocols" are tocols that have beta and delta dimethyl moieties on the benzene ring of the chroman moiety, and "β-tocols" are tocols that have alpha and delta dimethyl moieties on the benzene ring of the chroman moiety. A "non-α-tocol" is a tocotrienol or tocopherol that is not α-tocotrienol or α-tocopherol, respectively. Examples of non-α-tocopherol include δ- tocopherol, γ- tocopherol, β tocopherol-, ε- tocopherol, ζ- tocopherol, η- tocopherol and their or an acceptable salt or ester thereof. "Pharmaceutically acceptable salts or ester" means salts or esters which possess the desired pharmacological activity and which are neither biologically nor otherwise undesirable.

Chemical derivatives and homologues of tocols, non-α-tocols and polyphenols are also acceptable for use in the invention, including pharmaceutically or dietary or nutritionally acceptable esters and salts. The terms "dietary" and "nutritionally" can be used interchangeably. Examples of tocol esters include acetate, succinate and palmitate. Examples of tocol salts include Na⁺, K⁺, NH₄⁺, Mg⁺², Al⁺³, Ca⁺², or amine salts.

In one embodiment the compositions of the invention comprise at least one source (a) of non-α-tocotrienol and/or non-α-tocopherol, and are preferably rich in δ- or γ-tocols, the δ-or γ-tocotrienol content or δ- or γ-tocopherol content or non-α-tocol content of the tocol source preferably being at least 0.1 % by weight of the source, preferably at least 1 %, more preferably greater than 2%, most preferably at least 7%, and optionally at least 9%, by weight of the source.

In another embodiment of the invention the non-α-tocol content of the tocol source (a) is at least 25% by weight, and preferably at least 50% by weight of that source. Optionally the tocol component of the composition consists essentially of (e.g. at least 90%, preferably at least 95% by weight of), or consists of, δ- or γ-tocotrienol and/or δ- or γ-tocopherol. Individual or mixed non-α-tocols can be obtained in substantially pure form (e.g. >_90% purity, preferably >_95% purity) by chemical synthesis or by purification from natural sources, according to conventional procedures.

Tocol sources which are especially rich in non-α-tocols include cereals (barley, oats, rice, wheat, rye, amaranths) and plant oils, such as rice bran oil, barley oil, olive oil and palm oil. The source may be enriched in non-α-tocols by conventional processing means, such as that disclosed in WO 91/17985. Preferred sources of δ-tocols for use in the invention are the Tocotrienol Rich Fractions (TRF; e.g. Nutriene® , TocoTab P40® , Gold-TriE® , Palmvitee® ), obtained by molecular distillation from any of the above-named plant oils. Normally, the δ-tocol source, which may be a fraction or extract, will comprise a mixture of fat-soluble molecules, including a range of tocopherols and tocotrienols. Optimally, the total tocol content of the tocol source is at least 30% by weight, preferably at least 50% by weight, and most preferably at least 70% by weight of the source. In order to reach this concentration of tocols it may be necessary to further concentrate commercially available tocol products such as TRF.

In one embodiment of the invention the weight ratio of total tocotrienols to α-tocopherol in the composition is greater than 0.5, preferably greater than 1.0, or even greater than 2.0 or 3.0. Optionally, the composition is substantially free of α-tocopherol, meaning that less than 5%, preferably less than 2% by weight of the total tocol content is α-tocopherol.

In another embodiment of the invention, source (a) is selected on the basis that the weight ratio of δ-tocol to α-tocopherol is greater than 0.65, preferably at least 1.0. One such source is TocoTab P40® (Fuji), a TRF prepared from palm oil by a process which selectively enriches in δ-tocotrienol. As an example of a δ-tocol source with a very high ratio of δ-tocol to α-tocopherol (> 1.5), Covi-Ox ^{TM}T-30 P (a mixture of tocopherols sold by Cognis GmbH), may be mentioned. By selecting source (a) to have a high ratio of δ-tocol to α-tocopherol it is possible to ingest a relatively large dose of antioxidant δ-tocols without the risk of exceeding the maximum daily intake of α-tocopherol recommended by national health authorities.

Suitable daily doses of total tocol in the composition of the invention are up to 2000 mg, preferably 5 to 1000mg, and optimally in the range of 50 to 500mg, generally 100-300mg. In a unit dosage, the composition of the invention preferably comprises 0.1 to 50mg, more preferably 0.25 to 20mg, and most preferably 0.5 to 15mg δ-tocol. The preferred daily dosages of non-α-tocol are up to about 1000mg, optionally 0.1 to 500mg, more preferably 0.25 to 125mg, usually 0.5 to 60mg, and optionally 5 to 250mg. Where TRF is used as the source (a) of non-α-tocol it is preferably consumed in an amount of 10 to 500mg/day, more preferably 50-250mg/day, and most preferably 20-100mg/day. In terms of body weight, an appropriate daily dosage range is about 5µg to 5mg non-α-tocol/kg, especially about 0.1 to 2mg non-α-tocol/kg. The daily dosage may correspond to a single unit dosage, or may be provided through multiple unit dosages.

Antioxidant source (b) of the composition of the invention can be defined as comprising at least one source or substance comprising polyphenolic compounds. These polyphenolics contribute to the exceptional antioxidant activities of sources (a) and (b) when used in combination.

In antioxidant source (b) there may be a single polyphenolic source or mixtures of different polyphenolic sources. The term "polyphenol" can be defined as a compound which possesses an aromatic ring bearing one or more hydroxy substituents, including functional derivatives and or an acceptable salt or ester thereof. Antioxidant agents containing polyphenols include plant, algal or fungal extracts or fractions rich in polyphenols, including flavonoids (isoflavones, anthocyanins, proanthocyanidins and anthocyanidins, flavans, flavones, flavones and flavanones). Specific examples of bioflavonoids are catechins (catechin, epicatechin, gallocatechin, epigallocatechin, epicatechin gallate, epigallocatechin gallate), quercetin, rutin, hesperidin and genistein.

Chemically synthesized or purified polyphenols and mixtures thereof may be used in place of plant extracts. Polyphenols may be synthesized or extracted from natural sources by any suitable method known to those skilled in the art, particularly using food-grade solvents. Liquid and solid (e.g. granulate or powder form) extracts are suitable.

As a rule, the greater the content of polyphenols in the extract the larger the synergistic effect observed. Therefore in one embodiment of the invention the antioxidant source (b) used in the invention is rich in polyphenols, i.e. having a polyphenol content of at least 50% by dry weight, preferably at least 65% by dry weight, and optionally at least 75% by dry weight of source (b). Antioxidant source (b) may consist essentially of polyphenols.

In particular, herbal infusions, teas, tisanes and extracts (e.g. lyophilised extracts) made from leaves of various sorts are acclaimed for their polyphenol-associated free-radical scavenging effects. Herbal sources from which antioxidant preparations can be made include materials obtained from: tea (*Camellia sinensis* and *Camellia assaimica*), rooibos, honeybush, *Ginkgo biloba, Phyllanthus* species, *Catechu gambir, Urtica* species, rosemary, sage, mint, etc. *Camellia sinensis* leaves are especially preferred. Green tea is particularly potent, as is red tea, white tea and Mohnai tea, but black tea, Oolong tea, yellow tea and jasmine tea may also be used. All varieties of Pu Erh teas and green teas are preferred. Where the source of polyphenols is leaf material, this material may be freshly-gathered leaves, fresh leaves that are dried immediately after gathering, fresh tea leaves that have been heat treated before drying to inactivate any enzymes present, unfermented tea, fermented tea, instant tea, tea solids, partially fermented tea leaves, and hot or cold aqueous and alcoholic extracts (tinctures) of these leaves, and mixtures thereof.

Polyphenols naturally occurring in *Camellia* plants and extracts therefrom are especially preferred for use as component (b) in the compositions of the invention. Included in this group of polyphenols are the catechins, namely epicatechin, epigallocatechin, epicatechin gallate, and epigallocatechin gallate. Sugar salts, sugar esters and other edible physiologically available derivatives of catechins may substitute for the naturally-occurring forms.

Effective extracts (e.g. aqueous or alcoholic) as a source of antioxidant (b) can be obtained from plant parts including leaves (teas), raw or cooked whole fruit, berries and vegetables, nuts (e.g. kola nuts) the skins of fruit, fruit flesh (e.g. orange, prune, tangerine, grapefruit, grape), fruit rind (e.g. citrus fruits), peel, pips, cones (e.g. hops), seeds (e.g. cocoa beans; coffee beans; *Silybum marianum*) or stones, bark, buds (e.g. *Syzygium aromaticum*), flowers (e.g. cruciferous vegetables), roots, rhizomes and tubers (e.g. *Curcuma longa, Taraxacum officinale, Arctium lappa, Glycyrrhiza glabra, Zingiber officinalis*), stalks, and so on. Grape seed extract is one preferred antioxidant-rich source (b), being rich in proanthocyanidins. Grape skin and grape juice (or red wine extract), maritime pine bark, apples, plums, cherries, red cabbage, wolfberry, hawthorn berry, bilberry, huckleberry, cranberry, elderberry and blueberry and other fruit and vegetables with red, blue or purple pigmentation are other sources of proanthocyanidins. Vegetables which are rich in polyphenols include peppers (e.g. chili peppers), spinach, broccoli, brussels sprouts, cabbage, kale, radishes, turnip and watercress. Extracts made from fruits and vegetables may have the advantage of providing both water-soluble vitamins (C and B complex) and polyphenols.

In another embodiment of the invention a unit dosage of the composition comprises 1 mg to 500mg polyphenols as antioxidant (b), in pure form or in an extract. Daily doses are suitably at least 1 mg, preferably 10mg to 2000mg, more preferably 100mg to 1000mg, and most preferably 250mg to 700mg.

In another embodiment of the invention the composition of the polyphenolic antioxidant source (b) is ideally present in equal weight (or concentration) or in weight excess (concentration excess) relative to the total tocol content of the composition, for example in a weight ratio of at least 1:1, preferably at least 1.5:1, more preferably at least 3:1, and most preferably at least 6:1.

In another embodiment of the invention, the dry weight ratio of polyphenols to δ-tocols in the composition of the invention is in the range 1:1 to 200: 1, more preferably 10:1 1 to 100:1, and most preferably 25:1 to 50:1.

In another embodiment, when δ-tocotrienol is the sole δ-tocol in the composition of the invention, antioxidant source (b) may comprise citrus flavonoids, but will in addition comprise other polyphenol(s).

Without wishing to be bound by theory, one possible explanation for the synergy observed using the compositions of this invention is that there is a cascade of redox cycles occurring in the serum. According to this theory, oxidation of endogenous α-tocopherol in the LDL complexes spares oxidation of LDL. Other tocols, especially non-α-tocols, regenerate α-tocopherol at the expense of being oxidized themselves. Oxidized tocols may be recycled by polyphenolic antioxidants or their metabolites, and only when these antioxidants are exhausted, after an extended lag phase, does irreversible LDL oxidation start to occur.

The observation that non-α-tocols such as δ-tocopherol or γ-tocopherol, are preferable to α-tocopherol isomers suggests that the single methyl group on the phenol ring of the delta tocols is an important factor in this activity. If the recycling theory is correct, the implication is that the chemistry of the non-α-tocols allows them to reduce LDL α-tocopherol, or to be reduced by water-soluble antioxidants, in a more efficient manner than other tocols, or to undergo more redox cycles.

The antioxidant activity of α-tocopherol is not restricted to prevention of LDL oxidation, and it is generally known as a protective vitamin for lipids, and particularly membrane phospholipids. Therefore, the composition of the invention can be used to support and supplement endogenous α-tocopherol antioxidant activity in diverse sites and tissues around the body. The composition can be administered in nutritional, pharmaceutical or cosmetic form. Although enteral administration (especially oral) is preferred, topical application is also envisaged, whether as a pharmaceutical salve or as a skin lotion or cosmetic.

In another embodiment, components (a) and (b) are provided together in pharmaceutical form or as a dietary supplement, in combination with a pharmaceutically acceptable carrier. As an alternative, each component can be provided separately, e.g. in kit form, for separate, simultaneous or sequential administration. The pharmaceuticals or supplements are compositions for enteral (oral, nasal, or rectal), parenteral or topical administration, and can be in liquid or solid form.

In yet another embodiment typical formulations will comprise (in % by weight) for example, from 0.001 % to 100%, preferably about 0.1 % to 50% by weight of each of active ingredients (a) and (b). Optionally the only active antioxidant components of the pharmaceutical or dietary supplement are tocol source (a) and antioxidant source (b). In some cases the tocol source (a) and antioxidant source (b) together constitute >10%, preferably >25%, and more preferably >50% by weight of the supplement; Pharmaceutical compositions and dietary supplements can be produced in the form of hard or soft (gel) capsules, tablets, dragees, sachets, powders, lozenges, syrups, liquid suspensions, emulsions and solutions in convenient dosage forms.

Oral pharmaceutical or dietary supplement forms can be made by conventional compounding procedures known in the pharmaceutical art, that is, by mixing the active substances together with pharmaceutically acceptable solid or liquid carriers and/or excipients, e.g. fillers such as cellulose, lactose, sucrose, mannitol, sorbitol, and calcium phosphates and binders, and binders such as starch, gelatin, tragacanth, methylcellulose and/or polyvinylpyrrolidone (PVP). A "dietary supplement" shall mean a product that is intended to supplement the human diet and is typically provided in the form of a pill, capsule, tablet or like formulation. The terms "dietary" and "nutritional" can be used interchangeably. Optional additives include lubricants and flow conditioners (e.g. silicic acid, silicon dioxide, talc, stearic acid, magnesium/calcium stearates, polyethylene glycol (PEG) diluents, disintegrating agents (e.g. starch, carboxymethyl starch, cross-linked PVP, agar, alginic acid and alginates) colouring agents, flavouring agents, and melting agents. Dragee cores are provided with suitable, optionally enteric, coatings, there being used inter alia concentrated sugar solutions which may contain gum arabic, talc, PVP, PEG and/or titanium dioxide, or coating solutions in suitable organic solvents or solvent mixtures or, for the preparation of enteric coatings, solutions of suitable cellulose preparations, such as acetylcellulose phthalate or hydroxypropylmethylcellulose phthalate. Dyes or pigments may be added to the tablets or dragee coatings, for example for identification purposes or to indicate different doses of active ingredient.

Other orally administrable pharmaceutical compositions are hard gelatin capsules and also soft, sealed capsules consisting of gelatin and a plasticiser, such as glycerol or sorbitol. The hard gelatin capsules may comprise the active ingredient in the form of granules, for example in admixture with fillers, such as lactose, binders, glidants and, if desired, stabilisers. In soft capsules the active ingredients are preferably dissolved or suspended in suitable liquids, such as fatty oils, paraffin oil or liquid polyethylene glycols; it is likewise possible to add stabilisers.

Since the composition comprises hydrophilic and hydrophobic compounds a conventional emulsifier or surfactant may be employed to disperse the active ingredients uniformly within the end-product. Optionally, the hydrophobic tocols can be encapsulated in vesicles or micelles for incorporation into low-fat or water-based spray-dried powders containing tocotrienols for compression moulding is described in WO 00/27393 and is incorporated herein by reference. Where fats or oils are used as solvents, the water-soluble polyphenolic antioxidant may need to be treated to ensure dispersion, for example by ensuring the particle size is small, and/or by preparing an emulsion using a surfactant having a HLB (Hydrophilicity-Lipophilicity Balance) below 10.

When the composition of the invention is externally applied for cosmetic, hair care or skin care/skin protective purposes, it can be in the form of a cream, gel, ointment, solid stick, lotion or a solution, optionally together with emulsifiers. Typical compositions include lotions containing water and/or alcohols and emollients such as hydrocarbon oils and waxes, silicone oils, vegetable, animal or marine fats or oils, glyceride derivatives, fatty acids or fatty acid esters, alcohol or alcohol ethers, lanolin and derivatives, polyhydric alcohols or esters, wax esters, sterols, phospholipids and the like. Conventional water-insoluble or aqueous bases for ointments and creams may be used: e.g. fluid paraffin, petrolatum, wool wax, oleyl oleate, and the like. Cetylstearylalcohol and other emulsifying alcohols may be employed, as can non-alcohol emulsifiers such as Tween 20, Tween 80, and lecithin. If desired, a compound that reduces penetration of, or absorbs, UV radiation can be included in the composition, some examples being PABA, Ti0₂, Zn0₂. In one embodiment a representative topical preparation will contain 0.1 to 20% by weight tocols, and at least an equivalent amount by weight of an antioxidant source (b).

The synergistic compositions of the invention can readily be incorporated into nutritional formulations, typically nutraceuticals or functional food and beverage products. For example, a non-α-tocol source can be added to a familiar food product which is naturally rich in polyphenols, some examples being: green tea, herbal teas, coffee, cocoa, grape juice, elderberry juice, citrus juices, berry juices, red wine, beer, and chocolate. Alternatively, purified polyphenols or a source of polyphenols are added to a conventional food or beverage product which is rich in tocols, particularly δ- or γ-tocopherols, such as cooking oils, margarines, whole grains, cereals and wholemeal products. Suitable product formats include yellow fat spreads, salad dressings, muesli, granola, muesli bars, crackers, biscuits, and cookies. Another option is to incorporate sources of both synergistic partners in a type of food or beverage which is low in endogenous polyphenols and δ-tocols, such as dairy products (cheese, butter, milk, milkshakes, yogurt, smoothies, ice-cream, cream) and desserts, non-dairy toppings, sorbets, icings etc.). In general, food and beverage products of the invention will comprise nutritional quantities of one or more of carbohydrates, protein, fibre and fat.

In another embodiment the polyphenol content of a finished liquid product (drink) will normally lie in the range of about 0.01 to 1 % by weight of the product, preferably 0.025 to 0.5% by weight. In a solid product (a food or reconstitutable drink powder) the usual range for polyphenol content is 0.05% to 5%, preferably 0.1 to 2%, by weight.

In another embodiment edible or drinkable products of the invention will generally contain 0.001 to 0.5%, preferably 0.01 % to 0.25% by weight of the finished product of tocol source (a).

The beverage compositions of the present invention may be manufactured and sold for consumption by the consumer in the form of a syrup, an aqueous concentrate, a dry powder, or (effervescent) granules which are diluted with water to yield a beverage, or as a tablet to be taken with water.

Food and drink products containing the combination of non-α-tocol source (a) and antioxidant source (b) are not only attractive to the consumer for their associated health benefits, but also have the advantageous characteristic of having a long shelf-life due to their potent antioxidant content. Accordingly, there may be no necessity to add artificial preservatives to the food and drink products of the invention, or at any rate amounts of these additives may be kept to a minimum. Fat- and oil-containing food products are particularly vulnerable to spoiling through the development of rancidity, and are therefore prime targets for inclusion of the synergistic antioxidant composition of the invention, optionally in conjunction with α-tocopherol.

If desired, other active ingredients may be combined with the tocol/ antioxidant source (b) composition of the invention. Particularly preferred are any natural ingredient or medicament known to have a beneficial effect in lowering cholesterol levels or in combating heart disease. It may, however, be advisable to exclude beta-carotene from the composition due to concerns about carcinogenicity. Other desirable components of the composition are factors that support or supplement free radical quenching, such as certain minerals, vitamins and metabolites.

The edible products of the invention optionally comprise conventional food additives, such as any of: emulsifiers, stabilizers, sweeteners, flavourings, colouring agents, preservatives, chelating agents, osmotic agents, acidulants, thickeners, texturisers, and so on.

The composition of the invention may be administered with beneficial effects to prevent, slow the progression of, or treat any disease associated with free radical damage, especially damage resulting in lipid peroxidation. This category includes CHD, rheumatism and inflammation, hepatitis, alcoholism, Alzheimer's disease, dementia, diabetes, multiple sclerosis, HIV and AIDS, collagen degradation, cataracts, macular degeneration, accelerated aging, neuropathies, myopathies, ischemia-reperfusion injury, haemorrhagic shock, gum disease, cold sores, psoriasis, eczema, seborrhea. The invention can also be applied to counteract the effects of formation of reactive oxygen species (ROS), whatever the cause, including that resulting from environmental pollution or irradiation, and from treatment with neoplastic drugs.

In particular, this new form of treatment can be applied to prevent or treat CHD and its underlying causes. Because of the direct connection between prevention of LDL oxidation and retardation of atherosclerosis, stroke patients and cardiac infarct patients, or those at risk of these conditions, will especially profit from treatment with the claimed compositions. Type 2 diabetics have an increased preponderance of small dense LDL, which predisposes to the oxidation of LDL, and in addition have lower levels of endogenous α-tocopherol. Therefore this patient group is a target group particularly well suited to treatment in accordance with the present method.

Ingestion of the composition of the invention can be employed as a cosmetic treatment method, in particular to counteract the development of wrinkles, delay skin aging, and improve the overall appearance of skin, eyes and hair.

Smokers and sunbathers are particularly vulnerable to cancer and aging of the skin and other tissues as a consequence of free radical generation. To some extent they can compensate by maintaining an elevated intake of antioxidants in their diets, which is easily achievable by consuming the composition of the present invention.

Exercise is believed to trigger an increase in free radical production by an increase in oxygen generation in the mitochondrial electron transport chain or by an increase in metalcatalysed free radical production due to mechanical and morphological damage to muscles. It has been reported that lipid and protein oxidation is a consequence of extended exercise. The present invention provides a convenient method for avoiding or minimizing oxidation damage consequent upon physical activity.

In accordance with the present invention, a method of preventing or treating a medical condition caused by generation of free radicals in the mammalian body comprises administering, to a person in need of such treatment, an effective amount of a combination of (a) a tocol source comprising non-α-tocols and (b) an antioxidant source comprising polyphenols, wherein non-α-tocols preferably constitute greater than 2% by weight of the source (a). According to another aspect of the invention, a method of preventing oxidative tissue damage in a mammal due to physical activity comprises administration of a tocol source comprising δ-tocols (preferably greater than 2% by weight δ-tocol) in combination with a polyphenol-containing antioxidant source.

### Examples

### Example 1

### Formulations of tocopherols and flavonoids

Various concentrations of tocopherols and flavonoids were tested for the combined ability to ameliorate disruption of energy metabolism secondary to stress. Oxidatively competent WIF B liver cells, obtained from Johns Hopkins University, were used in the cell viability assays. Tocopherols were obtained from Sigma Chemical Co. and stock solutions (about 40 mg/ml) prepared by mixture with 10 mM glycocholic acid. Glycocholic acid alone does not protect cells at the concentrations used.

In experiments carried out in support of the invention, various concentrations of flavonoids and tocopherols were tested for synergistic ability to ameliorate disruption of energy metabolism secondary to stress. WIF B cells, cultured according to methods known in the art (US Patent 6,232,060), were treated simultaneously with diosmin, an exemplary flavonoid, γ-tocopherol and antimycin A. The cells were treated with various low concentrations of diosmin (Sigma Chemical Co., cat. # D3525) and various low concentrations of tocopherol. Cell viability was measured 4 hours after treatment using SYTOX Measurement.

SYTOX Green nucleic acid stain is a high-affinity nucleic acid stain that easily penetrates cells with a compromised plasma membrane but will not cross the membrane of live cells. The nucleic acids of dead cells fluoresce bright green at 524nm when excited with the 488nm spectral source or with any other 450-500nm source. Hence during a time course with cells incubated with SYTOX, the fluorescent emission at 524nm is proportional to cell death. In the cell viability assay chemical ischemia is created in cells that are incubated with the SYTOX dye. The compound antimycin is used to simulate ischemia at the same time as stimulating the cells with forskolin. Antimycin inhibits electron transport from FADH2 between complexes II and III and hence lowers cellular ATP synthesis. The assay is performed on cells grown in 96-well format allowing high throughput screening of compounds.

Materials: (i)SYTOX Green dye, supplied as a 5 mM solution in DMSO (Molecular Probes # S-7020) is stored at -20°C until use. ii.) Antimycin-A: (Sigma # A-8674) dissolve 5.28 g in 10ml of DMSO to make a 10mM stock solution. Aliquot at 100µl. This 10mM stock is then diluted to 1mM with DMSO and aliquoted at 25 µL. Both the 10mM and the 1mM are stored at -20°C until use. (iii) Forskolin (Sigma # F-6886), dissolve 50mg aliquot in 1.62ml of DMSO to make a 100mM stock solution. Aliquot at 100µl and store at -20°C until use. iv.) HBSS-SYTOX-FORSKOLIN-ANTIMYCIN (H-S-F-A) solution: H-S-F-A solution is made up fresh prior to each assay in sterile 175ml or 225ml Falcon bottles. The working concentrations for the assay are 3 µM SYTOX, 50µM Forskolin and 30µM Antimycin.
In each experiment, a 1: 500 dilution with HBSS from the 500 µM stock solution is made to generate a 1 µM final working solution of SYTOX Green. The final concentration of DMSO is 0.2%.

Cells were grown in 96-well black plates (with or without clear bottoms) at a density of 1000 cells per well for 24 hours. The media was carefully removed from the cells and replaced with 200µl of 1x HBSS. The plates were then placed in the humidified 37°C incubators of the Biomek 2000 Side Loader. Cells were then washed with 1x HBSS prior to addition of the H-S-A-compound test solution, as described below.
Compound(s) were mixed with the 1 µM SYTOX working solution and added to the washed cell culture wells. Fluorescence was measured at designated time points (488 nm excitation and 530 nm emission), using as blank cell-free wells only containing HBSS buffer with SYTOX green dye. The data was compared to non-treated cells (control) and expressed as mean + S.D. from 4 culture wells. SYTOX Green fluorescence units obtained from each group were corrected for basal fluorescence (cell-free wells with only HBSS and fluorescence reagent). A paired t-test was performed comparing treated groups (Toxin + Agent) with non-treated groups (Toxin only) after subtracting the control groups (HBSS only) for each data set. Comparisons among groups were statistically evaluated by two-tailed t-test (Graphpad Prism Software, version 2.0). Differences are considered significant at P < 0.05.

The experiments demonstrated that diosmin and γ-tocopherol act synergistically. At concentrations ranging from 0.03-0.3 µg/ml, γ-tocopherol was not protective by itself; however, in the presence of diosmin (33-100 µM), synergism was observed. As noted above, diosmin by itself was not effective over this range. Lower concentrations of diosmin (3.3-11 µM ) were also synergistic with 0.1 µg/ml γ-tocopherol. Optimized formulations were also revealed (for example, 0.03µg/ml γ-tocopherol plus 100 µM diosmin, 1 µg/ml γ-tocopherol plus 11 µM diosmin).

Various concentrations of the flavonoid hesperetin and various tocopherols were tested for ability to ameliorate disruptions in energy metabolism secondary to stress.
Oxidatively competent cells were treated simultaneously with hesperetin, antimycin A, and (+)-δ-tocopherol or (+)-γ-tocopherol. Cells were treated and cell death was recorded four hours later. At the concentrations used (2 and 50 µM hesperetin, 0.22 and 3.6 µg/ml tocopherol), hesperetin and tocopherols were generally ineffective when tested separately. Addition of 50 µM hesperetin to various tocopherols greatly reduced cell death in a synergistic manner. For example, cell death was reduced to 20% with the combination of 50 µM hesperetin and 0.22 µg/ml (+)-γ-tocopherol. Cell death was reduced to 30% with the combination of 50 µM hesperetin and 0.22 µg/ml (+)-δ-tocopherol. The combination of 0.22 µg/ml tocopherol with hesperetin in the range of 2-60 µM was also particularly synergistic. Thus, hesperetin acts synergistically with various tocopherols, including, without limitation, δ- and γ-tocopherol.
Additional experiments determined the optimal formulation comprising γ-tocopherol and biochanin A. Compositions comprising 0.11 or 0.33 µg/ml γ-tocopherol and 15 µg/ml biochanin A allowed a nearly 40% decrease in cell death compared to γ-tocopherol alone.

## Claims

1. A dietary supplement composition comprising a mixture of:
(a) a source of non-α-tocol or an acceptable salt or ester thereof comprising from 2% to 95% by weight non-α-tocol; and
(b) polyphenol or an acceptable salt or ester thereof,
with the proviso that when (b) comprises citrus flavonoids, (a) comprises δ-tocopherol or a salt or ester thereof.

2. A dietary supplement composition according to claim 1, wherein a unit dosage comprises 0.5 to 50mg of non-α-tocol or a salt or ester thereof.

3. A daily dosage dietary supplement composition comprising:
(a) from 5mg to 1000mg of
(i) non-α-tocopherol or a salt or ester thereof or
(ii) a mixture of non-α-tocotrienol or a salt or ester thereof and non-α-tocopherol or a salt or ester thereof; and
(b) polyphenol or a salt or ester thereof.

4. A daily dosage dietary supplement composition comprising:
(a) from 0.1 to 500mg of
(i) non-α-tocopherol or a salt or ester thereof or
(ii) a mixture of non-α-tocotrienol or a salt or ester thereof and non-α-tocopherol or a salt or ester thereof; and
(b) polyphenol or a salt or ester thereof.

5. A daily dosage dietary supplement composition comprising:
(a) from 5 to 250mg of
(i) non-α-tocopherol or a salt or ester thereof or
(ii) a mixture of non-α-tocotrienol or a salt or ester thereof and non-α-tocopherol or a salt or ester thereof; and
(b) polyphenol or a salt or ester thereof.

6. A daily dosage dietary supplement composition comprising:
(a) from 5mg to 1000mg of
(i) γ-tocopherol or a salt or ester thereof or
(ii) a mixture of δ-tocopherol or a salt or ester thereof and γ-tocopherol or a salt or ester thereof; and
(b) polyphenol or a salt or ester thereof.

7. A dietary supplement composition according to any preceding claim wherein the dry weight ratio of (b):(a) is in the range of 1:1 to 200:1.

8. A dietary supplement composition according to any preceding claim wherein the dry weight ratio of (b): (a) is in the range of 10:1 to 100:1.

9. A dietary supplement composition according to any preceding claim wherein the dry weight ratio of (b):(a) is in the range of 25:1 to 50:1.

10. A dietary supplement composition according to any preceding claim which comprises one or more flavonoids.

11. A dietary supplement composition according to any one of claims 5-9 which comprises one or more catechins.

12. A dietary supplement composition according to any preceding claim wherein the weight ratio of δ-tocol or a salt or ester thereof to non-α-tocol or an acceptable salt or ester thereof is not greater than 25% of said composition.

13. A dietary supplement composition according to any of claims 1 to 12 which is substantially free of α-tocopherol or a salt or ester thereof.

14. A dietary supplement composition according to claim 13 which is substantially free of tocols other than δ-tocols or a salt or ester thereof.

15. A dietary supplement composition according to any preceding claim which comprises a single polyphenolic antioxidant source.

16. A nutritional product comprising the composition of any of claims 1 to 15.

17. A food or beverage product comprising:
(a) a source of non-α-tocol or an acceptable salt or ester thereof comprising from 2% to 95% by weight non-α-tocol or a salt or ester thereof; and
(b) an antioxidant source comprising polyphenol or an acceptable salt or ester thereof, and optionally one or more of carbohydrate, fat, fibre and protein.

18. A pharmaceutical composition or dietary supplement comprising the composition of any of claims 1 to 15, and a pharmaceutically acceptable carrier.

19. A pharmaceutical composition or dietary supplement according to claim 18, wherein the antioxidant components of the composition or supplement consist essentially of (a) and (b).

20. Use of a dietary supplement composition according to any of claims 1 to 15 as a scavenger of free radicals.

21. Use of a dietary supplement composition comprising
(a) a source of non-α-tocol or an acceptable salt or ester thereof comprising from 2% to 95% by weight non-α-tocol or a salt or ester thereof; and
(b) an antioxidant source comprising polyphenol or an acceptable salt or ester thereof, as a preservative for preventing spoilage of food and beverage products.

22. A dietary supplement composition according to any of claims 1 to 15 for use as a medicament.

23. Use of a dietary supplement composition according to any of claims 1 to 15 in the manufacture of a medicament or nutritional formulation for the prevention or treatment of a disease condition or corporal damage associated with the generation of free radicals.

24. Use according to claim 23 where said disease condition is arteriosclerosis, rheumatism, or cancer.

25. Use according to claim 23 wherein said corporal damage is caused by physical activity, exposure to the sun, or smoking.

26. A kit comprising:
(a) a source of non-α-tocol or an acceptable salt or ester thereof comprising from 2% to 95% by weight non-α-tocol or a salt or ester thereof; and
(b) an antioxidant source comprising polyphenol or an acceptable salt or ester thereof, for separate, sequential or simultaneous administration.

27. A cosmetic composition comprising:
(a) a source of non-α-tocol or an acceptable salt or ester thereof comprising from 2% to 95% by weight non-α-tocol; and
(b) an antioxidant source comprising polyphenol or an acceptable salt or ester thereof; and
(c) a cosmetic base.
